# EUROPEAN PATENT SPECIFICATION

(11) **EP 3 571 496 B1**
(45) Date of publication and mention of the grant of the patent: **18.11.2026**
(21) Application number: 17837893.1
(22) Date of filing: 27.12.2017
(51) Int. Cl.: G01N 27/12, G01N 33/00, B82Y 25/00, B82Y 30/00

(54) **RESISTIVE VOC GAS SENSOR COMPRISING POLYMERIC COMPOSITE MATERIAL AND USE THEROF**
WIDERSTANDSBASIERTER VOC-GASSENSOR AUS EINEM POLYMEREN KOMPOSITMATERIAL UND DESSEN VERWENDUNG
CAPTEUR DE COV À RÉSISTANCE COMPRENANT UN MATÉRIAU COMPOSITE POLYMÈRE ET SON UTILISATION

(30) Priority: 27.12.2016 GB 201622299; 13.06.2017 GB 201709372
(43) Date of publication of application: 27.11.2019
(73) Proprietor: Lussey, David, Richmond, North Yorkshire DL10 7QL (GB)
(72) Inventor: Lussey, David, Richmond, North Yorkshire DL10 7QL (GB)
(74) Representative: Elsworth, Dominic Stephen
(86) International application number: PCT/GB2017/053893
(87) International publication number: WO 2018/122556

(56) References cited:
- EP-A1- 0 699 964
- WO-A1-2016/207667
- WO-A1-2017/103592
- GB-A- 2 539 697
- US-A- 4 586 143
- US-A- 5 541 570
- US-A1- 2010 025 238
- RAZZAQ ET AL: "Thermal, electrical and magnetic studies of magnetite filled polyurethane shape memory polymers", MATERIALS SCIENCE AND ENGINEERING: A, ELSEVIER, AMSTERDAM, NL, vol. 444, no. 1-2, 9 December 2006 (2006-12-09), pages 227 - 235, XP005799932, ISSN: 0921-5093, DOI: 10.1016/J.MSEA.2006.08.083
- WEIDENFELLER B ET AL: "Thermal and electrical properties of magnetite filled polymers", COMPOSITES PART A: APPLIED SCIENCE AND MANUFACTU, ELSEVIER, AMSTERDAM, NL, vol. 33, no. 8, 6 September 2002 (2002-09-06), pages 1041 - 1053, XP004379755, ISSN: 1359-835X, DOI: 10.1016/S1359-835X(02)00085-4
- BERTOCCI FRANCESCO ET AL: "Fast nano-grained Fe3O4 gas sensor for the control of CO and NO2 concentrations", 2016 IEEE METROLOGY FOR AEROSPACE (METROAEROSPACE), IEEE, 22 June 2016 (2016-06-22), pages 286 - 290, XP032967710, DOI: 10.1109/METROAEROSPACE.2016.7573228
- SEONG SOO AN ET AL: "Core-shell nanostructured hybrid composites for volatile organic compound detection", INTERNATIONAL JOURNAL OF NANOMEDICINE, 28 August 2015 (2015-08-28), AUCKLAND, NZ, pages 203, XP055469748, ISSN: 1176-9114, DOI: 10.2147/IJN.S88305
- GRAHAM A ET AL: "Metal-polymer composite sensors for volatile organic compounds: Part 2. Stand alone chemi-resistors", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 177, 24 November 2012 (2012-11-24), pages 507 - 514, XP028964082, ISSN: 0925-4005, DOI: 10.1016/J.SNB.2012.11.046

## Description

### Field of the Invention

This invention relates to an electric charge storage medium containing shaped particles of conductive metals and/or metal oxides. Such particles can be made in a composite form using a binder and the mechanical and electrical qualities of the binder can be used synergistically with selected particulate filler morphology and amounts to produce a super-sensitive control charge composite storage medium which, when charged can be made to respond selectively to sense various stimuli and also provides an indication of the onset and amount of the stimulus by rate of change of the charge stored within the composite.

### Background of the Invention

Electrically conductive polymer composites which exhibit changing electrical resistance when subject to compressive or tensile forces are known. Also known are sensing composites which can be charged to respond to volatile organic compounds (VOCs) but these composites are erratic in operation having charge characteristics which cannot be controlled to provide real time repeatable resolution of the stimulus data.

Razzaq et al, "Thermal, electrical and magnetic studies of magnetite filled polyurethane shape memory polymers', Materials Science and Engineering: A, vol. 444, no. 1-2, pages 227-235 and Weidenfeller B et al, 'Thermal and electrical properties of magnetic filled polymers', Composites Part A: Applied Science and Manufacturing, vol. 33 (2002), no. 8, pages 1041-1053 both describe polymer binders containing magnetite.

GB2539697 A discloses a polymer composition comprising at least one substantially non-conductive polymer binder and at least first and second electrically conductive fillers, wherein the first electrically conductive filler is comprised of particles having a void bearing structure, and the second electrically conductive filler is comprised of particles which are substantially spherical in shape. Preferably, the second electrically conductive filler is comprised of magnetite.

### Summary of the Invention

According to the invention there is provided the use of a polymer composite as an electrical charge storage medium for volatile organic compound (VOC) sensing as set out in Claim 1. The medium is comprised of an electrically conductive filler within a non-conductive binder composition and wherein the amount of charge stored by the medium may be controlled by changing the relative amounts of electrically conductive filler to non-conductive binder and/ or by adjusting the relative proportions, shape and size of the electrically conductive fillers.

According to a further aspect of the invention there is provided a volatile organic compound (VOC) sensor as set out in Claim 11.

An electrical charge storage medium comprises a quantity of first electrically conductive particles contained in a non conductive binder, which first electrically conductive particles are magnetite particles, wherein the quantity of magnetite particles includes a distribution of particle sizes between sub-micron and tens of microns, and wherein the magnetite particles have a plurality of planar faces, adjacent planar faces connected at a vertex, the particles each having a plurality of vertices wherein the magnetite particles are irregular in shape and have a low aspect ratio.

Preferably, the shape of the first electrically conductive particles in the distribution fall under the particle shape definitions of, "oblate", that is tabular, and/or "bladed", that is a flat or elongated shape form.

Preferably, the distribution of particles the particle size of the first electrically conductive particles at d₅₀ is between 50 and 75 micron

Preferably, the distribution of particles the particle size of the first electrically conductive particles at d₅₀ is between 60 and 65 micron.

Preferably, in the distribution of particles the particle size of the first electrically conductive particles at d₅₀ is be between 20 and 25 micron.

Preferably,, in the distribution of particles the particle size of the first electrically conductive particles size at d₅₀ is be between 5 and 15 micron.

Preferably, particle size of the first electrically conductive particles at d₅₀ is around 10 micron.

Preferably, the distribution of particle sizes between sub-micron and tens of microns in the quantity of magnetite particles includes sub-micron sized particles and particles that are tens of microns in size.

The electrical charge storage medium may further comprise a second type of electrically conductive particle of a different shape to the first electrically conductive particle.

Advantageously, the second type of electrically conductive particle has one of the following shapes: void bearing, plate like, needle like and spherical.

The second type of electrically conductive particle may be selected from the group comprising: silver, nickel, copper, iron, oxides of silver, nickel copper and iron including but not limited to magnetite and synthetic magnetite.

The particle size of the second type of electrically conductive particles may be between 10 and **15** micron.

The binder is a polymer binder.

Preferably, the polymer is one of: a silicone polymer, a polyurethane polymer; polyethylene and an acrylic polymer.

Advantageously, the binder forms a thin layer around each particle or agglomerations of particles in the particle distribution.

Preferably, the thickness of the thin layer is in the order of 10 nano-metres.

It is preferred that the particles and/or agglomerations of particles are coated with the thin layer of binder and wherein adjacent separate coated particles and/or agglomerations of particles are in contact with each other and wherein the charge storage medium is formed by a matrix of so coated particles and/or agglomerations of particles.

Advantageously, the proportion of magnetite particles to binder is greater than or equal to 33% by weight.

The proportion of magnetite particles to binder may be greater than or equal to 50% by weight.

The proportion of magnetite particles to binder may be greater than or equal to 66% by weight.

The proportion of magnetite particles to binder may be greater than or equal to 75% by weight.

The proportion of magnetite particles to binder may be greater than or equal to 83% by weight.

The proportion of magnetite particles to binder may be greater than or equal to 90%.

According to a second aspect of the invention (not claimed) there is provided a method of manufacturing an electrical charge storage medium according to the first aspect of the invention, the method comprising the step of:
mixing the electrically conductive particles with the binder until the mixture thereof is homogeneous.

The method of manufacturing an electrical charge storage medium may further comprise mixing of the electrically conductive particles with the binder is low shear mixing, the mixture being mixed by a low shear paddle or by folding.

The method of manufacturing an electrical charge storage medium may include the additional step of heating the binder to reduce the viscosity thereof, prior to and/or during the step of mixing the electrically conductive particles with the binder.

The method of manufacturing an electrical charge storage medium may include the additional step of drying the resulting mixture.

The method of manufacturing an electrical charge storage medium may include the additional steps of: specifying the ratio of first electrically conductive particles contained in a non conductive binder so that the resulting mixture has a granular and/or frangible crumb structure where some granules are connected to one another. The method may include the further step of milling the resulting mixture to break apart a frangible crumb structure into coated granules with a mill that is configured to break apart connected granules. Typically, the parts of the mill acting on the frangible club structure are spaced apart.

The method may include the further step of applying the mixture to a non-conductive substrate.

The method may comprising the further step of drying the mixture.

The proportion of magnetite to binder may be greater than or equal to 75% or 83% by weight. The resulting mixture may comprise a frangible crumb structure.

According to a third aspect of the invention there is provided a VOC sensor comprising an electrical charge storage medium of the first aspect of the invention and spaced apart electrodes attached to the charge storage medium, and wherein the space defined by the space between the electrodes and the extent of the electrodes defines an active area.

Preferably, the spaced apart electrodes extend into the electrical charge storage medium by a distance.

The charge storage medium may be applied to a non-conductive substrate and wherein the spaced apart electrodes are attached to a top surface of the charge storage medium.

Preferably, the charge storage medium is applied to the non-conductive substrate in a thickness of between 50 and 250 micron.

Preferably, the charge storage medium is applied to the non-conductive substrate in a thickness of between 100 and 150 micron.

The sensor further comprises a voltage source connected to the spaced apart electrodes.

The voltage may be applied for a specific time period or continuously and is either polarised or not polarised

The voltage source may be configured to apply a voltage at a level that causes the electrical charge storage medium to lock into a state of conduction.

The sensor **is** a volatile organic compound (VOC) sensor.

The non-conductive binder may be sensitive to a selected volatile organic compound or family of volatile organic compounds.

The electrical charge storage medium may be adapted to be sensitive to at least one of the volatile organic compounds including acetone.

The sensor may further comprise a measuring device attached to the electrodes and configured to measure a parameter indicative of resistance.

Preferably, the measuring device is an ohm meter or an ammeter.

The sensor may further comprise a resetting means configured to reset the conductive state of the electrical charge storage medium, the resetting means including a switch arranged to connect selectively the voltage source to the electrical charge storage medium.

Aspect ratio in relation to a particle is the ratio of the width of the particle to its height. A low aspect ratio means that the height to width ration is approaching **1:1.**

Volatile organic compounds (VOCs) are organic chemicals that have a high vapour pressure at ordinary room temperature. Their high vapour pressure results from a low boiling point, which causes large numbers of molecules to evaporate or sublimate from the liquid or solid form of the compound and enter the surrounding air. VOCs are numerous and varied. They include both human-made and naturally occurring chemical compounds. For example, most scents or odors are of VOCs.

There are many reasons why it may be desirable to sense VOCs. Some are very harmful to humans, animal and plant health and are regulated by law. Others are known to be symptomatic of certain health conditions. For example, acetone on the breath can be associated with diabetes.

It would therefore be desirable to provide an improved sensor capable of sensing the presence of VOC's.

It would be desirable to provide an improved sensor capable of determining a concentration of VOC present in a sample.

It would be desirable to provide an improved sensor capable of sensing the presence of and/or the concentration of a specified VOC, for example acetone.

### Brief Description of the Drawings

In the Drawings:
Figure 1a is a micrograph of a distribution of particles of magnetite of Example 1;
Figure 1b is a micrograph of a distribution of particles of magnetite of Example 1 after a milling process;
Figure 2a is a micrograph of a distribution of particles of magnetite of Example 2;
Figure 2b is a micrograph of a distribution of particles of magnetite of Example 2 after a milling process;
Figure 3a is a micrograph of a distribution of particles of magnetite of Example 3;
Figure 3b is a micrograph of a distribution of particles of magnetite of Example 3 after a milling process;
Figure 4a is a side view of a sensor according to an aspect of the invention;
Figure 4b is a plan view of the sensor illustrated in Figure 4b; and
Figure 5 is a schematic representation of a sensor according to another embodiment of the invention.

### Description of the Invention

The Magnif range of magnetite produced by LKAB of Sweden has been found to be particularly useful in the invention as it has been found to have unusual electrical qualities with low electrical noise and stable, controllable charge and discharge parameters.

A super-sensor can be produced by mixing different sizes of magnetite particles with a water-based polyurethane binder in quantities which exceed the normal percolation loading for magnetite. LKAB Magnif ranges of magnetite bound with Witcobond 781 water based polyurethane have been used to produce thermo-setting ink versions of the invention. Other types of thermoset and thermoplastic polymer binders may be used in these composites to enhance their physical, chemical or electrical response to different types of analytes and forces.

Other types of conductive/semi-conductive particles and particle shapes including other sources of magnetite as well as different polymer binders can be used in this invention, alone or in combination, including with the LKAB magnetite materials, to produce different types of sensing capability, response, sensitivity and charge characteristics in the final composite. Mixing the components of the composite is done using a low shear paddle or folding regime until the mix just achieves homogeneity. Mixing is then complete and the polymer component can be cross-linked/set if required for immediate use or stored for later use.

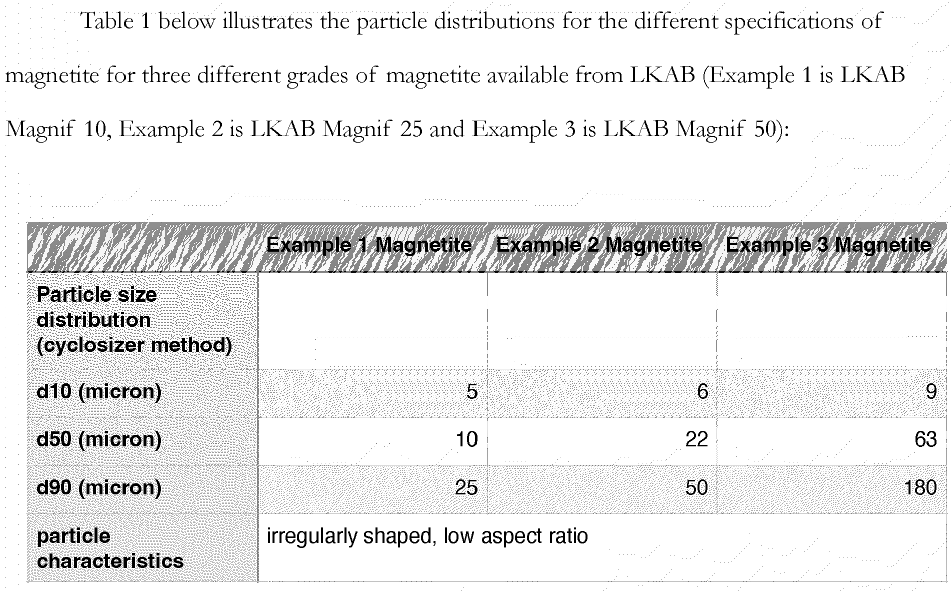

Chemical analysis of three grades of magnetite from the Magnif range produced by LKAB os Sweden are set out in Table 2. As can be seen, the material in all example is over 98% Fe3O4.

**Table 2:**

| Typical Values for LKAB Magnetite | | | | |
|---|---|---|---|---|
| MAGNIF | 10 Example 1 | 25 Example 2 | 50 Example 3 | |
| | | | | |
| Fe304 | 98,7 | 98,4 | 98,7 | % |
| MnO | 0,05 | 0,05 | 0,05 | % |
| CaO | 0,05 | 0,06 | 0,05 | % |
| MgO | 0,24 | 0,25 | 0,23 | % |
| Al2O3 | 0,22 | 0,22 | 0,2 | % |
| Si02 | 0,16 | 0,27 | 0,11 | % |
| Ti02 | 0,35 | 0,33 | 0,25 | % |
| V2O5 | 0,26 | 0,27 | 0,24 | % |
| K20 | 0,004 | 0,003 | 0,004 | % |
| S | 32 | 32 | 32 | ppm |
| Cl water sol | 10 | 10 | 10 | ppm |
| Fe | 71,4 | 71,2 | 71,4 | % |
| | | | | |
| Particle density | 5,2 | 5,2 | 5,2 | g/cm3 |
| Bulk density | around 2-3 g/cm3 | | | |

Charge is applied to the finished composite by applying a voltage through electrodes in contact with the composite. A DC voltage of as low as 30 volts has been found to impart a charge to the composite. A DC voltage of 50 volts may be used. A piezo spark generator has also been used to achieve a higher charged state and in all cases only low currents are required. The effect of applying charge is to change the quiescent, very low-conduction state of the composite, to become more conductive. This makes the composite more responsive to forces or the presence of analytes. Charge can be imparted in a fraction of a second using either a low or high voltage and can be topped up periodically to replace lost charge if continuous sensing is required. 'Look-through' sensing, similar to radar, is also possible with periodic charging.

These polymer compositions may be useful in the manufacture of sensors capable of sensing, VOCs, ionizing radiation, non-ionizing radiation, temperature, force, pressure, chemical species, and biological species. For example, a particular biological species may be known to release certain VOC(s). A chemical species when heated may release certain VOC(s). A polymer binder known to absorb the certain VOC(s) will react and increase the resistance of the charged composite. The polymer binder may expand and increase the resistance of the charged composite when contacted by certain VOC(s).

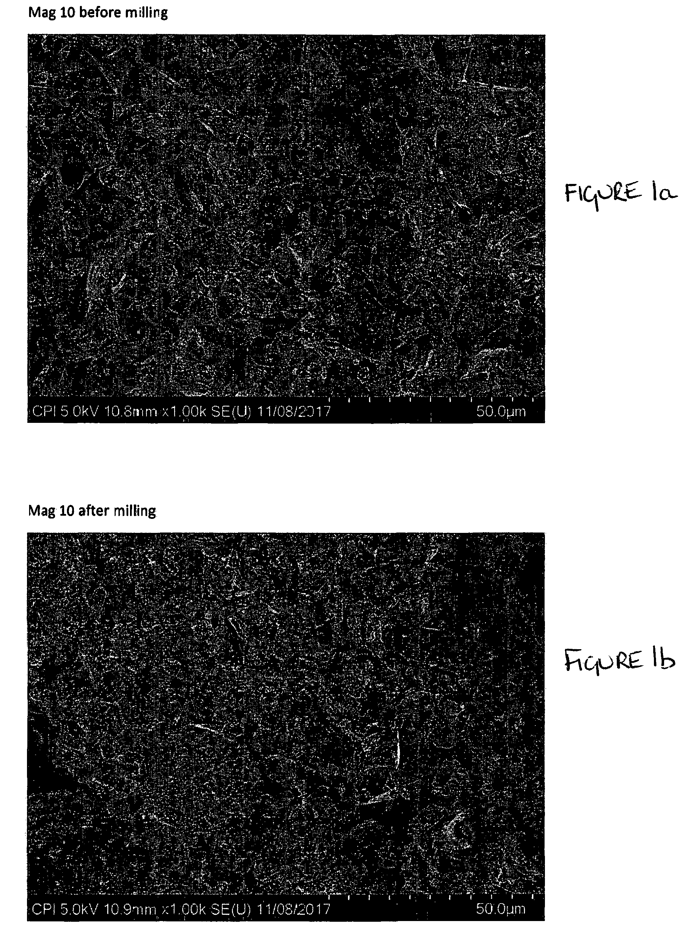

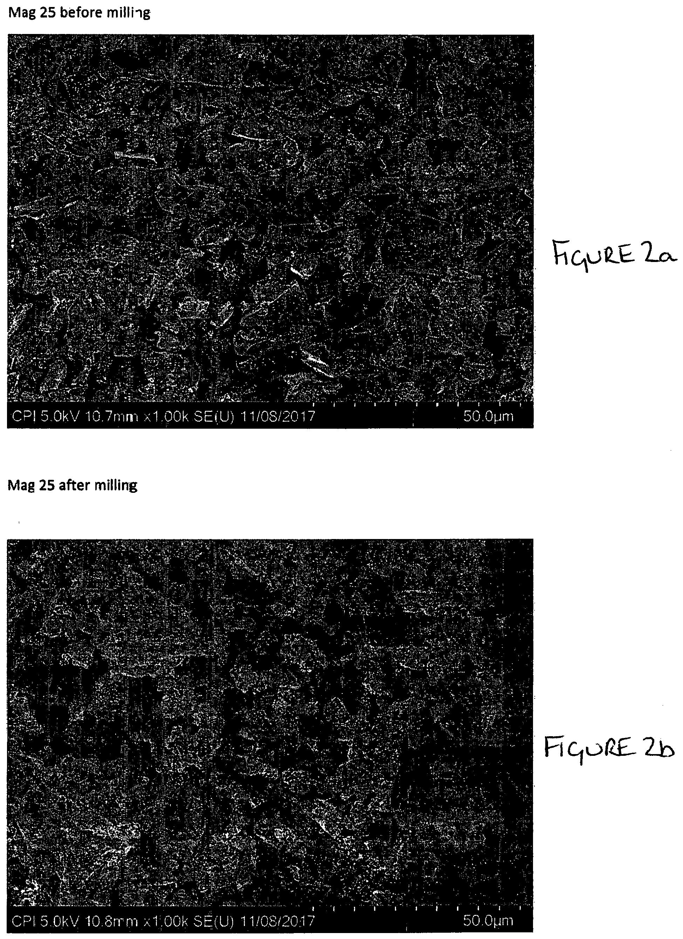

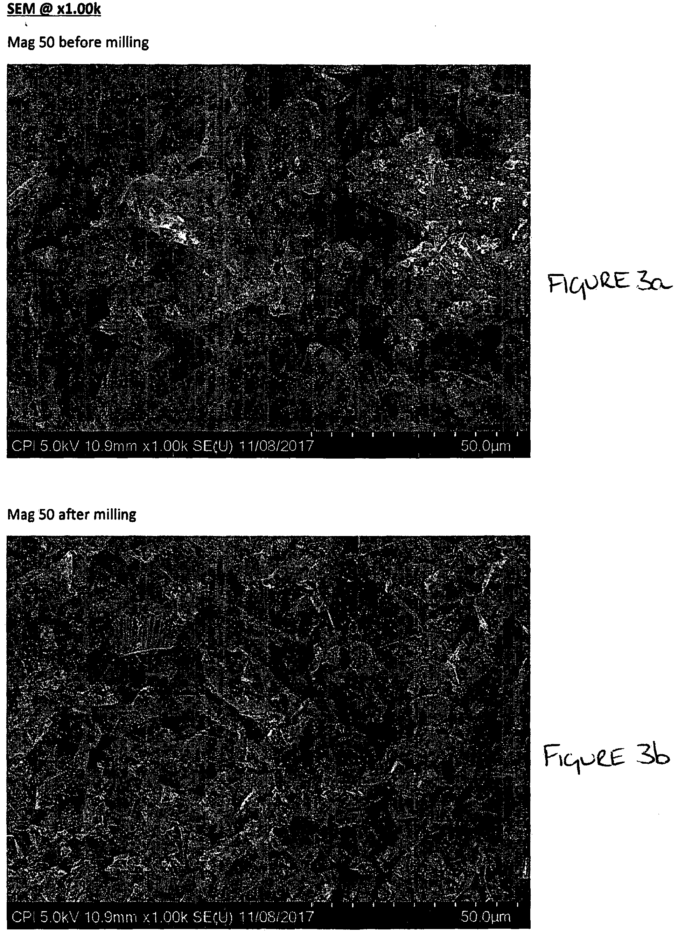

All 3 products of the LKAB magnetite range can be used in the invention but I will describe the Magnif 50 as it gives the most sensitive performance of the range. If required, sensitivity can be reduced by the addition of synthetic magnetite to the Magnif magnetite. Synthetic magnetite is typically substantially spherical in shape. Sensitivity can be increased by the addition of dendritic silver powder. Other metals of different shapes may also be utilised to change sensitivity. Sensitivity may also be changed by further comminuting and/or classification of the base Magnif material to remove particles in identified size ranges. Figures 1b, 2b and 3b are micro-graphs illustrating particle shapes and sizes after milling of three different size grades of magnetite. The micro-graphs of Figures 1a, 2a and 3a show the corresponding grade of magnetite prior to size reduction through milling. In each case when the milled magnetite is mixed with a polymer the resulting composite is less sensitive than a composite made by mixing the magnetite that has not been milled with the polymer, that is sensitivity increases with increasing particle size. In this instance the term, "sensitivity" means that the magnitude of change in resistance resulting from an external input, such as the presence of a VOC, a bidder change in resistance meaning that the composite material is more sensitive.

To make a sensing composite LKAB magnetite Magnif 50 was functionalised by coating it with Witcobond 781 liquid polymer thinned down with 2 parts water to 1 part 781 polymer. This ratio can be changed and effects recovery, sensitivity and background noise levels in the finished composite. Dry Magnif 50 magnetite was added to the polymer/water solution in the ratio of 3 parts of magnetite to 1 part polymer/water solution by weight. The mix is stirred using low shear mixer until it just becomes homogenous as seen by a sheen on the surface of the mix. The mixing is complete at that point and the product can be used in the wet form as a coating or printing ink and then dried with the application of heat, blown air or microwaves as the 781 polymer is a thermoset. Other polymers can be used and polymers with specified absorption levels to specific VOC analytes can be chosen to provide VOC selectivity to the sensor. Where the polymer is a polyurethane for example the composite provides a large resistance change to acetone.

Where it is desired to create granules comprising single or agglomerated particles of magnetite the amount of polymer used is decreased when compared to the ratios described above, which will produce a solid material when cool and dry. The ratio of magnetite to polymer is increase to 6 parts of magnetite to 1 part of polymer water solution by weight made as described above using the same type of magnetite and polymer, that is 1 part to polymer to 2 parts water. By reducing the amount of polymer the resulting mixture is very dry and may be described as a frangible crumb. Where adjacent granules are connected together they can be broken apart by passing the mixture through a mill that is arranged to break apart joined granules rather than reduce the size of individual granules. For example, a mill comprising rollers that are arrange with a small space therebetween could be used. It may be of advantage to use rollers having a relatively soft surface, such as rubber, rather than steel or other rollers. Nevertheless, the method of producing a granular form of the composite material of the invention is not limited to any particular type of mill.

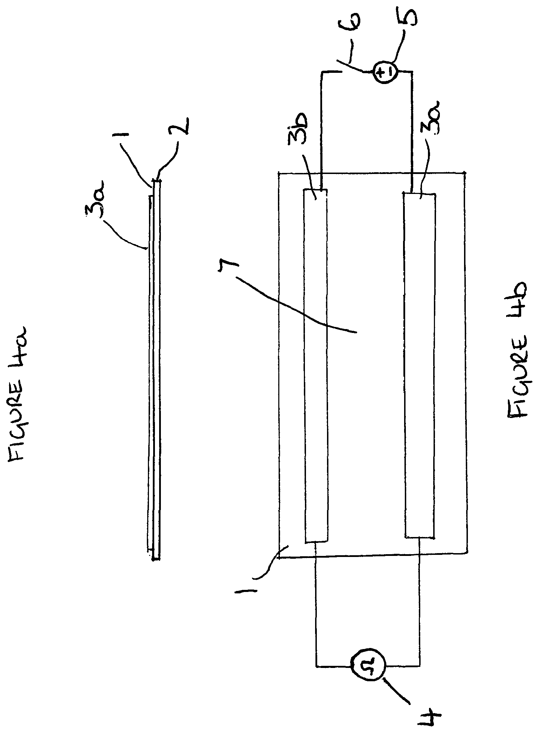

A sensor is illustrated in Figures 4a and 4b. The illustrated sensor comprises a layer 1 of composite ink comprising the charge storage medium of the invention applied to the surface of a non-conductive layer of material 2. The non-conductive material may be plastic, film, paper, card, textile or foam for example. Where the non-conductive material has a three dimensional structure, such as a textile or foam, the composite ink may be applied the structure thereof.

In its simplest form a sensor will comprise a 100 to 150 micron coating of the ink on a non-conductive surface. Where the material has a three dimensional structure, such as a textile or foam, the composite ink may be applied the structure thereof.

Two conductive electrodes 3a, 3b are attached to the top surface of the dried ink with a conductive adhesive. These electrodes are arranged to have a gap between them which forms the area 7 sensitive to the analyte VOC. The width of the gap can be from 100 micron to a few mm and its dimensions form the active area of the sensor. The length of the gap in the electrodes can be from 100 microns to many centimetres depending on the area requiring exposure to the analyte.

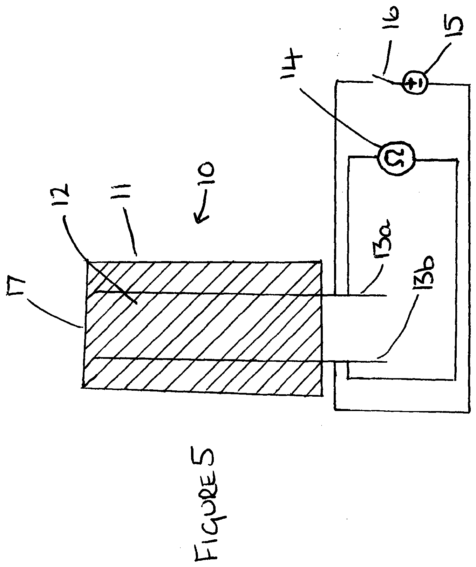

Figures 5 illustrates an alternative embodiment of a sensor. The sensor 10 of Figure 5 comprises a non-conductive container 11 filled with a composite material of electrical charge storage medium 12 indicated by the cross-hatched lines. Electrodes 13a, 13b extend through a bottom wall of the container 11 into the charge storage medium. An area 17 sensitive to analyte is formed at an open end of the container. The charge storage medium may be in a number of forms. For example, the charge storage medium may be formed by pouring electrical charge storage medium into the container whilst medium is in a pourable state. The medium may be heated to reduce its viscosity to facilitate such pouring. The medium, once poured into the container 11 then sets over a period of time. Alternatively, the medium may be provided by filing the container with a matrix of granules, the granules being formed by coating individual particles and/or agglomerations of particles of magnetite with the selected polymer binder. In another alternative the medium may be carried on a foam carrier which is shaped and dimensioned to fit the container 11.

In the embodiments of Figures 4a, 4b and 5 the electrodes 3a, 13a, 3b, 13b are connected to an ohm meter 6, 16 for detecting a change resistance of the medium 1, 12 and a voltage source 5, 15. A switch 6, 16 is provided for connecting the voltage source 5, 15 to the electrodes 3a, 13a, 3b, 13b selectively. The voltage source is used to apply a charge to the medium causing a change its resistance.

The sensing composite, that is the electrical charge storage medium of the invention, has a quiescent resistance state of more than 10⁹ Ohms but surprisingly it has been found that the resistance can be reduced by the application of a voltage. This effect is immediately apparent on application of the voltage.

The voltage can be applied in a constant, pulsed, rising or falling form and does not need to be polarised. A low voltage produces a smaller effect which although transient, is related to the voltage level. A higher voltage produces a larger effect, that is the resistance of the material is reduced more. At a certain voltage level, this transient behaviour disappears and the composite stays in a locked holding state of conduction. It is this locked state that provides the composite with the best start condition for detecting the presence of a VOC. The VOC interacts with the polymer of the composite unlocking the holding state. It is believed that the VOC causes the polymer to swell initially unlocking the holding state. Once unlocked, the initial low resistance of the composite in the holding state increases towards the quiescent high resistance state whilst the sensor is exposed to an analyte. The response is surprisingly rapid and can cover more than 4 orders of resistance change in under 1 second. Also surprisingly, if the analyte is removed, the resistance change will stop within a second and return to a level very close to the charged start state within a few seconds. Alternatively, the composite can also be returned to its charged state instantly by applying a further charge. Whichever way the sensor achieves the charged state, it is immediately ready to detect an analyte.

### Test:

For the test, acetone vapour was blown from a rubber bulb onto the sensor from a distance of 5cm for 1 second.

The charge used for the Magnif 50 sensor detailed above was produced by applying a DC voltage pulse of 1Kv limited to 1mA for 100mS to the sensor which initially showed a resistance above measurable limits of 10⁹ Ohms

This resulted in an immediate locked state resistance in the sensor composite of 100KOhms.

The first exposure to Acetone produced an increase in resistance to more than 10⁹Ohms in less than 1 second. After removal of the vapour the sensor self-recovered to 550KOhms in 10 seconds.

A second test (as para1) was conducted immediately after the above-mentioned 10 second interval and the sensor showed an increase in resistance to more than 10⁹ Ohms in less than 1 second and self-recovered to 1.5MOhms in 10 seconds.

A third test produced a similar response but the return was to 2.7MOhms

A fourth test produced a similar response but the return was to 2.0MOhms

A fifth test produced a similar response but the return was to 2.4MOhms.

A test was then conducted on the same sensor by applying a 1Kv charge to reset the sensor after each acetone activation. This test was conducted 5 times and the sensor went into an immediate locked state of 100KOhms on each occasion after a sensing activation. The interval between activations was 30 seconds and on each occasion the resistance to the exposure was above 10⁹ Ohms.

## Claims

1. The use of a polymer composite (1, 12) as an electrical charge storage medium for volatile organic compound (VOC) sensing, the polymer composite comprising a quantity of first electrically conductive particles contained in a non conductive polymer binder, which first electrically conductive particles are magnetite particles, wherein the quantity of magnetite particles includes a distribution of particle sizes between sub-micron and tens of microns, and wherein the magnetite particles have a plurality of planar faces, adjacent planar faces connected at a vertex, the particles each having a plurality of vertices wherein the magnetite particles are irregular in shape and have a low aspect ratio, wherein the non conductive polymer binder forms a thin layer around magnetite particles of the quantity thereof such that said magnetite particles are separated from one another by said non-conductive polymer binder in which they are contained, the polymer composite having spaced apart electrodes (3a, 13a, 3b, 13b) attached thereto, **characterised in that** the electrodes are connected to a voltage source (5, 15) of 30 volts or greater, and wherein without a voltage across the electrodes the polymer composite has a quiescent resistance state of more than 10⁹ Ohms and wherein with the voltage applied across the electrodes to said polymer composite the resistance thereof is reduced.

2. The use of a polymer composite as a charge storage medium according to Claim 1, wherein in the distribution of particles the particle size of the first electrically conductive particles at d₅₀ is one of:
between 50 and 75 micron;
between 60 and 65 micron;
between 20 and 25 micron;
between 5 and 15 micron;
10 micron; and/or
wherein the distribution of particle sizes between sub-micron and tens of microns in the quantity of magnetite particles includes sub-micron sized particles and particles that are tens of microns in size.

3. The use of a polymer composite as a charge storage medium according to any preceding claim, further comprising a second type of electrically conductive particle of a different shape to the first electrically conductive particle.

4. The use of a polymer composite as a charge storage medium according to Claim 3, wherein:
the second type of electrically conductive particle has one of the following shapes: void bearing, plate like, needle like and spherical; and/or
the second type of electrically conductive particle is selected from the group comprising: silver, nickel, copper, iron, oxides of silver, nickel copper and iron including but not limited to magnetite and synthetic magnetite; and/or
the particle size of the second type of electrically conductive particles is between 10 and 15 micron.

5. The use of a polymer composite as a charge storage medium according to any preceding claim, wherein the polymer binder is one of: a silicone polymer binder, a polyurethane polymer binder, polyethylene binder and an acrylic polymer binder.

6. The use of a polymer composite as a charge storage medium according to any preceding, wherein:
the polymer binder forms a thin layer around agglomerations of particles in the particle distribution; and/or
the thickness of the thin layer is in the order of 10 nano-metres; and/or
wherein the particles and/or agglomerations of particles are coated with the thin layer of polymer binder and wherein adjacent separate coated particles and/or agglomerations of particles are in contact with each other and wherein the polymer composite is formed by a matrix of so coated particles and/or agglomerations of particles.

7. The use of a polymer composite as a charge storage medium according to any preceding claim, wherein the proportion of magnetite particles to polymer binder is one of:
greater than or equal to 33% by weight;
greater than or equal to 75% by weight;
greater than or equal to 90% by weight.

8. The use of a polymer composite as a charge storage medium according to any preceding claim, further comprising a resetting means configured to reset the conductive state of the polymer composite, the resetting means including a switch (6, 16) arranged to connect selectively the voltage source (5, 15) to the polymer composite (1, 12).

9. The use of a polymer composite as a charge storage medium according to Claim 8, wherein a charge is applied to the polymer composite via the electrodes by:
a direct current source connected to the electrodes; or
a piezo spark generator connected to the electrodes.

10. The use of a polymer composite as a charge storage medium according to Claim 8 or 9, wherein:
the voltage is applied by the voltage source for a specific time period or continuously in a pulsed rising or falling form and is either polarised or not polarised; or
the voltage is applied by the voltage source for a specific time period or continuously in a pulsed rising or falling form and is either polarised or not polarised and the voltage source is configured to apply a voltage at a level that causes the polymer composite to lock into a state of conduction.

11. A volatile organic compound (VOC) sensor (10) comprising a polymer composite (1, 12) as a charge storage medium, the polymer composite comprising a quantity of first electrically conductive particles contained in a non conductive polymer binder, which first electrically conductive particles are magnetite particles, wherein the quantity of magnetite particles includes a distribution of particle sizes between sub-micron and tens of microns, and wherein the magnetite particles have a plurality of planar faces, adjacent planar faces connected at a vertex, the particles each having a plurality of vertices wherein the magnetite particles are irregular in shape and have a low aspect ratio, wherein the non conductive polymer binder forms a thin layer around magnetite particles of the quantity thereof such that said magnetite particles are separated from one another by said non-conductive polymer binder in which they are contained, the polymer composite having electrodes (3a, 13a, 3b, 13b) attached thereto, **characterised in that** the sensor further includes a voltage source (5, 15) configured to apply 30 volts or greater in a pulsed form and the electrodes are connected to the voltage source, and wherein without a voltage across the electrodes the polymer composite has a quiescent resistance state of more than 10⁹ Ohms and wherein with the voltage applied across the electrodes to said polymer composite the resistance thereof is reduced, wherein said electrodes attached to the polymer composite (1, 12) are spaced apart, and wherein the space defined by the space between the electrodes and the extent of the said electrodes defines an active area.

12. A VOC sensor according to Claim 11, wherein the spaced apart electrodes (3a, 3b, 13a, 13b) extend into the polymer composite (1, 12) by a distance.

13. A VOC sensor according to Claim 11 or 12, wherein:
the polymer composite (1, 12) is applied to a non-conductive substrate (2, 11) and wherein the spaced apart electrodes (3a, 3b, 13a, 13b) are attached to a top surface of the polymer composite (1, 12); or
the polymer composite(1, 12) is applied to a non-conductive substrate (2, 11) and wherein the spaced apart electrodes (3a, 3b, 13a, 13b) are attached to a top surface of the polymer composite (1, 12) and the polymer composite (1, 12) is applied to the non-conductive substrate (2, 11) in a thickness of between 50 and 250 micron or between 100 and 150 micron.

14. A VOC sensor according to Claim 11, wherein:
the voltage is applied by the voltage source for a specific time period or continuously the voltage and is either polarised or not polarised; or
the voltage is applied by the voltage source for a specific time period or continuously and is either polarised or not polarised and the voltage source is configured to apply a voltage at a level that causes the polymer composite (1, 12) to lock into a state of conduction.

15. A VOC sensor according to any of claims 11 to 14, wherein the non-conductive polymer binder is sensitive to a selected volatile organic compound.

16. A VOC sensor according to any of claims 11 to 15, wherein the polymer composite (1, 12) is adapted to be sensitive to at least one of the volatile organic compounds including acetone.

17. A VOC sensor according to any of Claims 11 to 16, further comprising:
a measuring device attached to the electrodes (3a, 3b, 13a, 13b) and configured to measure a parameter indicative of resistance; or
a measuring device attached to the electrodes (3a, 3b, 13a, 13b) and configured to measure a parameter indicative of resistance and wherein the measuring device is an ohm meter or an ammeter.

18. A VOC sensor according to any of Claims 11 to 17, further comprising a resetting means configured to reset the conductive state of the polymer composite (1, 12), the resetting means including a switch 6, 16 arranged to connect selectively the voltage source (5, 15) to the polymer composite (1, 12).

## Patentansprüche

1. Verwendung eines Polymerverbundstoffs (1, 12) als ein elektrisches Ladungsspeichermedium zur Erfassung von flüchtigen organischen Verbindungen (VOC), wobei der Polymerverbundstoff eine Menge von ersten elektrisch leitenden Teilchen umfasst, die in einem nichtleitenden Polymerbindemittel enthalten sind, wobei diese ersten elektrisch leitenden Teilchen Magnetitteilchen sind, wobei die Menge von Magnetitteilchen eine Verteilung von Teilchengrößen zwischen Submikron und mehreren zehn Mikron einschließt, und wobei die Magnetitteilchen eine Vielzahl von planaren Flächen aufweisen, wobei benachbarte planare Flächen an einem Scheitelpunkt verbunden sind, wobei die Teilchen jeweils eine Vielzahl von Scheitelpunkten aufweisen, wobei die Magnetitteilchen eine unregelmäßige Form aufweisen und einen niedrigen Formfaktor aufweisen, wobei das nichtleitende Polymerbindemittel eine dünne Schicht um Magnetitteilchen der Menge dieser bildet, so dass die besagten Magnetitteilchen durch das besagte nichtleitende Polymerbindemittel, in dem sie enthalten sind, voneinander getrennt sind, wobei der Polymerverbundstoff voneinander beabstandete Elektroden (3a, 13a, 3b, 13b) aufweist, die daran angebracht sind, **dadurch gekennzeichnet, dass** die Elektroden mit einer Spannungsquelle (5, 15) von 30 Volt oder höher verbunden sind, und wobei der Polymerverbundstoff ohne eine Spannung über die Elektroden einen Ruhewiderstandszustand von mehr als 10⁹ Ohm aufweist und wobei bei über die Elektroden zu dem besagten Polymerverbundstoff angelegter Spannung der Widerstand dieses verringert ist.

2. Verwendung eines Polymerverbundstoffs als ein Ladungsspeichermedium nach Anspruch 1, wobei die Teilchengröße der ersten elektrisch leitenden Teilchen bei d₅₀ in der Verteilung von Teilchen eine der folgenden ist:
zwischen 50 und 75 Mikron;
zwischen 60 und 65 Mikron;
zwischen 20 und 25 Mikron;
zwischen 5 und 15 Mikron;
10 Mikron; und/oder
wobei die Verteilung von Teilchengrößen zwischen Submikron und mehreren zehn Mikron in der Menge von Magnetitteilchen Teilchen in Submikrongröße und Teilchen, die eine Größe von mehreren zehn Mikron aufweisen, einschließt.

3. Verwendung eines Polymerverbundstoffs als ein Ladungsspeichermedium nach einem vorhergehenden Anspruch, ferner umfassend einen zweiten Typ von elektrisch leitendem Teilchen mit einer anderen Form als das erste elektrisch leitende Teilchen.

4. Verwendung eines Polymerverbundstoffs als ein Ladungsspeichermedium nach Anspruch 3, wobei:
der zweite Typ von elektrisch leitendem Teilchen eine der folgenden Formen aufweist: hohlraumführend, plättchenartig, nadelartig und sphärisch; und/oder
der zweite Typ von elektrisch leitendem Teilchen aus der Gruppe ausgewählt ist, umfassend: Silber, Nickel, Kupfer, Eisen, Silberoxide, Nickel-Kupfer-Eisen, einschließlich Magnetit und synthetischem Magnetit, jedoch nicht darauf beschränkt; und/oder
die Teilchengröße des zweiten Typs von elektrisch leitenden Teilchen zwischen 10 und 15 Mikron liegt.

5. Verwendung eines Polymerverbundstoffs als ein Ladungsspeichermedium nach einem vorhergehenden Anspruch, wobei das Polymerbindemittel eines der folgenden ist: ein Silikon-Polymerbindemittel, ein Polyurethan-Polymerbindemittel, Polyethylen-Bindemittel und ein Acryl-Polymerbindemittel.

6. Verwendung eines Polymerverbundstoffs als ein Ladungsspeichermedium nach einem vorhergehenden Anspruch, wobei:
das Polymerbindemittel eine dünne Schicht um Agglomerationen von Teilchen in der Teilchenverteilung bildet; und/oder
die Dicke der dünnen Schicht in der Größenordnung von 10 Nanometer liegt; und/oder wobei die Teilchen und/oder die Agglomerationen von Teilchen mit der dünnen Schicht aus Polymerbindemittel beschichtet sind und wobei benachbarte getrennte beschichtete Teilchen und/oder Agglomerationen von Teilchen miteinander in Kontakt stehen und wobei der Polymerverbundstoff von einer Matrix von so beschichteten Teilchen und/oder Agglomerationen von Teilchen gebildet wird.

7. Verwendung eines Polymerverbundstoffs als ein Ladungsspeichermedium nach einem vorhergehenden Anspruch, wobei der Anteil von Magnetitteilchen zu Polymerbindemittel einer der folgenden ist:
größer als oder gleich 33 Gew.-%;
größer als oder gleich 75 Gew.-%;
größer als oder gleich 90 Gew.-%.

8. Verwendung eines Polymerverbundstoffs als ein Ladungsspeichermedium nach einem vorhergehenden Anspruch, ferner umfassend ein Rücksetzmittel, das dazu konfiguriert ist, den Leitungszustand des Polymerverbundstoffs zurückzusetzen, wobei das Rücksetzmittel einen Schalter (6, 16) einschließt, der dazu eingerichtet ist, die Spannungsquelle (5, 15) selektiv mit dem Polymerverbundstoff (1, 12) zu verbinden.

9. Verwendung eines Polymerverbundstoffs als ein Ladungsspeichermedium nach Anspruch 8, wobei eine Ladung an den Polymerverbundstoff mittels der Elektroden angelegt wird durch:
eine Gleichstromquelle, die mit den Elektroden verbunden ist; oder
einem Piezo-Funkengenerator, der mit den Elektroden verbunden ist.

10. Verwendung eines Polymerverbundstoffs als ein Ladungsspeichermedium nach Anspruch 8 oder 9, wobei:
die Spannung von der Spannungsquelle für einen spezifischen Zeitraum oder kontinuierlich in einer gepulsten ansteigenden oder abfallenden Form angelegt wird und entweder polarisiert oder nichtpolarisiert ist; oder
die Spannung von der Spannungsquelle für einen spezifischen Zeitraum oder kontinuierlich in einer gepulsten ansteigenden oder abfallenden Form angelegt wird und entweder polarisiert oder nichtpolarisiert ist und die Spannungsquelle dazu konfiguriert ist, eine Spannung auf einem Pegel anzulegen, der bewirkt, dass sich der Polymerverbundstoff in einem Leitungszustand fixiert.

11. Sensor (10) für flüchtige organische Verbindungen (VOC), umfassend einen Polymerverbundstoff (1, 10) als ein Ladungsspeichermedium, wobei der Polymerverbundstoff eine Menge von ersten elektrisch leitenden Teilchen umfasst, die in einem nichtleitenden Polymerbindemittel enthalten sind, wobei diese ersten elektrisch leitenden Teilchen Magnetitteilchen sind, wobei die Menge von Magnetitteilchen eine Verteilung von Teilchengrößen zwischen Submikron und mehreren zehn Mikron einschließt, und wobei die Magnetitteilchen eine Vielzahl von planaren Flächen aufweisen, wobei benachbarte planare Flächen an einem Scheitelpunkt verbunden sind, wobei die Teilchen jeweils eine Vielzahl von Scheitelpunkten aufweisen, wobei die Magnetitteilchen eine unregelmäßige Form aufweisen und einen niedrigen Formfaktor aufweisen, wobei das nichtleitende Polymerbindemittel eine dünne Schicht um Magnetitteilchen der Menge dieser bildet, so dass die besagten Magnetitteilchen durch das besagte nichtleitende Polymerbindemittel, in dem sie enthalten sind, voneinander getrennt sind, wobei der Polymerverbundstoff Elektroden (3a, 13a, 3b, 13b) aufweist, die daran angebracht sind, **dadurch gekennzeichnet, dass** der Sensor ferner eine Spannungsquelle (5, 15) einschließt, die dazu konfiguriert ist, 30 Volt oder mehr in einer gepulsten Form anzulegen, und wobei der Polymerverbundstoff ohne eine Spannung über die Elektroden einen Ruhewiderstandszustand von mehr als 10⁹ Ohm aufweist und wobei bei über die Elektroden zu dem besagten Polymerverbundstoff angelegter Spannung der Widerstand dieses verringert ist, wobei die besagten Elektroden, die an dem Polymerverbundstoff (1, 12) angebracht sind, voneinander beabstandet sind, und wobei der Raum, der von dem Raum zwischen den Elektroden definiert wird, und die Ausdehnung der besagten Elektroden einen aktiven Bereich definieren.

12. VOC-Sensor nach Anspruch 11, wobei sich die voneinander beabstandeten Elektroden (3a, 3b, 13a, 13b) um einen Abstand in den Polymerverbundstoff (1, 12) erstrecken.

13. VOC-Sensor nach Anspruch 11 oder 12, wobei:
der Polymerverbundstoff (1, 12) auf ein nichtleitendes Substrat (2, 11) aufgebracht wird und wobei die voneinander beabstandeten Elektroden (3a, 3b, 13a, 13b) an einer oberen Fläche des Polymerverbundstoffs (1, 12) angebracht werden; oder
der Polymerverbundstoff (1, 12) auf ein nichtleitendes Substrat (2, 11) aufgebracht wird und wobei die voneinander beabstandeten Elektroden (3a, 3b, 13a, 13b) an einer oberen Fläche des Polymerverbundstoffs (1, 12) angebracht werden und der Polymerverbundstoff (1, 12) auf das nichtleitende Substrat (2, 11) in einer Dicke zwischen 50 und 250 Mikron oder zwischen 100 und 150 Mikron aufgebracht wird.

14. VOC-Sensor nach Anspruch 11, wobei:
die Spannung von der Spannungsquelle für einen spezifischen Zeitraum oder kontinuierlich angelegt wird und die Spannung entweder polarisiert oder nichtpolarisiert ist; oder
die Spannung von der Spannungsquelle für einen spezifischen Zeitraum oder kontinuierlich angelegt wird und entweder polarisiert oder nichtpolarisiert ist und die Spannungsquelle dazu konfiguriert ist, eine Spannung auf einem Pegel anzulegen, der bewirkt, dass sich der Polymerverbundstoff (1, 12) in einem Leitungszustand fixiert.

15. VOC-Sensor nach einem der Ansprüche 11 bis 14, wobei das nichtleitende Polymerbindemittel für eine ausgewählte flüchtige organische Verbindung empfindlich ist.

16. VOC-Sensor nach einem der Ansprüche 11 bis 15, wobei der Polymerverbundstoff (1, 12) dazu eingerichtet ist, für mindestens eine der flüchtigen organischen Verbindungen empfindlich zu sein, einschließend Aceton.

17. VOC-Sensor nach einem der Ansprüche 11 bis 16, ferner umfassend:
eine Messvorrichtung, die an den Elektroden (3a, 3b, 13a, 13b) angebracht wird und dazu konfiguriert ist, einen Parameter zu messen, der einen Widerstand angibt; oder eine Messvorrichtung, die an den Elektroden (3a, 3b, 13a, 13b) angebracht wird und dazu konfiguriert ist, einen Parameter zu messen, der einen Widerstand angibt, und wobei die Messvorrichtung ein Ohmmeter oder ein Amperemeter ist.

18. VOC-Sensor nach einem der Ansprüche 11 bis 17, ferner umfassend ein Rücksetzmittel, das dazu konfiguriert ist, den Leitungszustand des Polymerverbundstoffs (1, 12) zurückzusetzen, wobei das Rücksetzmittel einen Schalter 6, 16 einschließt, der dazu eingerichtet ist, die Spannungsquelle (5, 15) selektiv mit dem Polymerverbundstoff (1, 12) zu verbinden.

## Revendications

1. L'utilisation d'un composite polymère (1, 12) en tant que support de stockage de charge électrique pour détecter un composé organique volatile (COV), le composite polymère comprenant une quantité de premières particules électriquement conductrices contenues dans un liant polymère non conducteur, lesquelles premières particules électriquement conductrices sont des particules de magnétite, dans laquelle la quantité de particules de magnétite inclut une distribution de tailles de particules allant du sous-micron à plusieurs dizaines de microns, et dans laquelle les particules de magnétite ont une pluralité de faces planes, les faces planes adjacentes étant reliées au niveau d'un sommet, les particule ayant chacune une pluralité de sommets dans laquelle les particules de magnétite sont de forme irrégulière et ont un faible rapport d'aspect, dans laquelle le liant polymère non conducteur forme une couche mince autour des particules de magnétite de la quantité de celles-ci de telle sorte que lesdites particules de magnétite sont séparées les unes des autres par ledit liant polymère non conducteur dans lequel elles sont contenues, le composite polymère ayant des électrodes espacées (3a, 13a, 3b, 13b) y étant fixées, **caractérisé en ce que** les électrodes sont reliées à une source de tension (5, 15) égale ou supérieure à 30 volts, et dans laquelle, sans une tension sur les électrodes, le composite polymère a un état de résistance au repos supérieur à 10⁹ Ohms et dans laquelle, avec la tension appliquée sur les électrodes audit composite polymère, la résistance de celles-ci est réduite.

2. L'utilisation d'un composite polymère en tant que support de stockage de charge selon la revendication 1, dans laquelle dans la distribution de particules, la taille de particule des premières particules électriquement conductrices à d₅₀ est soit :
entre 50 et 75 microns ;
entre 60 et 65 microns ;
entre 20 et 25 microns ;
entre 5 et 15 microns ;
10 microns ; et/ou
dans laquelle la distribution de tailles de particules allant du sous-micron à plusieurs dizaines de microns dans la quantité de particules de magnétite inclut des particules d'une taille inférieure au micron et des particules d'une taille de plusieurs dizaines de microns.

3. L'utilisation d'un composite polymère en tant que support de stockage de charge selon l'une quelconque des revendications précédentes, comprenant en outre un deuxième type de particule électriquement conductrice d'une forme différente de la première particule électriquement conductrice.

4. L'utilisation d'un composite polymère en tant que support de stockage de charge selon la revendication 3, dans laquelle :
le deuxième type de particule électriquement conductrice a une des formes suivantes : forme comportant des vides, forme de plaque, forme d'aiguille et forme sphérique et/ou
le deuxième type de particule électriquement conductrice est sélectionné dans le groupe comprenant : l'argent, le nickel, le cuivre, le fer, des oxydes d'argent, de nickel, de cuivre et de fer incluant mais sans être y limité la magnétite et la magnétite synthétique ; et/ou
la taille de particule du deuxième type de particule électriquement conductrice est entre 10 et 15 microns.

5. L'utilisation d'un composite polymère en tant que support de stockage de charge selon l'une quelconque des revendications précédentes, dans laquelle le liant polymère est soit : un liant polymère de silicone, un liant polymère de polyuréthane, un liant polyéthylène ou un liant polymère acrylique.

6. L'utilisation d'un composite polymère en tant que support de stockage de charge selon l'une quelconque des revendications précédentes, dans laquelle :
le liant polymère forme une couche mince autour d'agglomérations de particules dans la distribution des particules ; et/ou
l'épaisseur de la couche mince est de l'ordre de 10 nanomètres ; et/ou
dans laquelle les particules et/ou des agglomérations de particules sont revêtues avec la couche mince de liant polymère et dans laquelle des particules et/ou des agglomérations de particules revêtues séparées adjacentes sont en contact les unes avec les autres et dans laquelle le composite polymère est formé par une matrice de particules et/ou d'agglomérations de particules ainsi revêtues.

7. L'utilisation d'un composite polymère en tant que support de stockage de charge selon l'une quelconque des revendications précédentes, dans laquelle la proportion de particules de magnétite au liant polymère est soit :
égale ou supérieure à 33 % en poids ;
égale ou supérieure à 75 % en poids ;
égale ou supérieure à 90 % en poids.

8. L'utilisation d'un composite polymère en tant que support de stockage de charge selon l'une quelconque des revendications précédentes, comprenant en outre un moyen de réinitialisation configuré pour réinitialiser l'état de conduction du composite polymère, le moyen de réinitialisation incluant un commutateur (6, 16) agencé pour relier sélectivement la source de tension (5, 15) au composite polymère (1, 12).

9. L'utilisation d'un composite polymère en tant que support de stockage de charge selon la revendication 8, dans laquelle une charge est appliquée au composite polymère par les électrodes par :
une source de courant directe reliée aux électrodes ; ou
un générateur d'étincelles piézoélectrique relié aux électrodes.

10. L'utilisation d'un composite polymère en tant que support de stockage de charge selon la revendication 8 ou 9, dans laquelle :
la tension est appliquée par la source de tension pendant une durée spécifique ou en continu sous une forme pulsée montante ou descendante et, soit est polarisée, soit n'est pas polarisée ; ou
la tension est appliquée par la source de tension pendant une durée spécifique ou en continu sous une forme pulsée montante ou descendante et, soit est polarisée, soit n'est pas polarisée, et la source de tension est configurée pour appliquer une tension à un niveau qui amène le composite polymère à se verrouiller dans un état de conduction.

11. Un détecteur de composé organique volatile (COV) (10) comprenant un composite polymère (1, 12) en tant que support de stockage de charge, le composite polymère comprenant une quantité de premières particules électriquement conductrices contenues dans un liant polymère non conducteur, lesquelles premières particules électriquement conductrices sont des particules de magnétite, dans lequel la quantité de particules de magnétite inclut une distribution de tailles de particules allant du sous-micron à plusieurs dizaines de microns, et dans lequel les particules de magnétite ont une pluralité de faces planes, les faces planes adjacentes étant reliées au niveau d'un sommet, les particules ayant chacune une pluralité de sommets dans lequel les particules de magnétite sont de forme irrégulière et ont un faible rapport d'aspect, dans lequel le liant polymère non conducteur forme une couche mince autour des particules de magnétite de la quantité de celles-ci de telle sorte que lesdites particules de magnétite sont séparées les unes des autres par ledit liant polymère non conducteur dans lequel elles sont contenues, le composite polymère ayant des électrodes (3a, 13a, 3b, 13b) y étant fixées, **caractérisé en ce que** le détecteur inclut en outre une source de tension (5, 15) configurée pour appliquer 30 volts ou plus sous une forme pulsée et les électrodes sont reliées à la source de tension, et dans lequel, sans une tension sur les électrodes, le composite polymère a un état de résistance au repos supérieur à 10⁹ Ohms et dans lequel, avec la tension appliquée sur les électrodes audit composite polymère, la résistance de celles-ci est réduite, dans lequel lesdites électrodes fixées au composite polymère (1, 12) sont espacées, et dans lequel l'espace défini par l'espace entre les électrodes et l'étendue desdites électrodes définit une zone active.

12. Un détecteur de COV selon la revendication 11, dans lequel les électrodes espacées (3a, 3b, 13a, 13b) s'étendent dans le composite polymère (1, 12) d'une certaine distance.

13. Un détecteur de COV selon la revendication 11 ou 12, dans lequel :
le composite polymère (1, 12) est appliqué à un substrat non conducteur (2, 11) et dans lequel les électrodes espacées (3a, 3b, 13a, 13b) sont fixées à une surface supérieure du composite polymère (1, 12) ; ou
le composite polymère (l, 12) est appliqué à un substrat non conducteur (2, 11) et dans lequel les électrodes espacées (3a, 3b, 13a, 13b) sont fixées à une surface supérieure du composite polymère (1, 12) et le composite polymère (1, 12) est appliqué au substrat non conducteur (2, 11) dans une épaisseur entre 50 et 250 microns ou entre 100 et 150 microns.

14. Un détecteur de COV selon la revendication 11, dans lequel :
la tension est appliquée par la source de tension pendant une durée spécifique ou en continu la tension et, soit est polarisée, soit n'est pas polarisée ; ou
la tension est appliquée par la source de tension pendant une durée spécifique ou en continu et, soit est polarisée, soit n'est pas polarisée, et la source de tension est configurée pour appliquer une tension à un niveau qui amène le composite polymère (1, 12) à se verrouiller dans un état de conduction.

15. Un détecteur de COV selon l'une quelconque des revendications 11 à 14, dans lequel le liant polymère non conducteur est sensible à un composé organique volatile sélectionné.

16. Un détecteur de COV selon l'une quelconque des revendications 11 à 15, dans lequel le composite polymère (1, 12) est adapté pour être sensible à un ou plusieurs des composés organiques volatiles incluant l'acétone.

17. Un détecteur de COV selon l'une quelconque des revendications 11 à 16, comprenant en outre :
un appareil de mesure fixé aux électrodes (3a, 3b, 13a, 13b) et configuré pour mesurer un paramètre indiquant la résistance ; ou
un appareil de mesure fixé aux électrodes (3a, 3b, 13a, 13b) et configuré pour mesurer un paramètre indiquant la résistance et dans lequel l'appareil de mesure est un ohmmètre ou un ampèremètre.

18. Un détecteur de COV selon l'une quelconque des revendications 11 à 17, comprenant en outre un moyen de réinitialisation configuré pour réinitialiser l'état de conduction du composite polymère (1, 12), le moyen de réinitialisation incluant un commutateur 6, 16 agencé pour relier sélectivement la source de tension (5, 15) au composite polymère (1, 12).
